# EUROPEAN PATENT APPLICATION

(11) **EP 4 555 884 A1**
(43) Date of publication of application: **21.05.2025**
(21) Application number: 22951142.3
(22) Date of filing: 14.07.2022
(51) Int. Cl.: A24F 40/51, A24F 40/20

(54) **AEROSOL GENERATION SYSTEM AND INFORMATION PROCESSING METHOD**

(71) Applicant: Japan Tobacco Inc., Tokyo 105-6927 (JP)
(72) Inventor: FUJIKI, Takashi, Tokyo 130-8603 (JP)
(74) Representative: Hoffmann Eitle
(86) International application number: PCT/JP2022/027690
(87) International publication number: WO 2024/013928

(57) **Abstract**

[Problem] To provide a mechanism capable of further improving the quality of user experience.

[Solution] Provided is an aerosol generation system provided with: a storage unit that has an internal space and an opening through which the internal space is communicated with the outside; at least one detection unit that emits light toward the internal space and detects received reflected light; and a control unit that determines whether or not an article inserted into the storage unit is a base material containing an aerosol source on the basis of the intensities of a plurality of reflected lights detected by the at least one detection unit.

## Description

### Technical Field

The present disclosure relates to an aerosol generating system and an information processing method.

### Background Art

An inhaler device that generates material to be inhaled by a user, such as an electronic tobacco and a nebulizer, is widely used. For example, an inhaler device uses a substrate including an aerosol source for generating an aerosol, a flavor source for imparting a flavor component to the generated aerosol, and the like, to generate an aerosol with the imparted flavor component. The user is able to taste a flavor by inhaling the aerosol with the imparted flavor component, generated by the inhaler device. An action that the user takes to inhale an aerosol is also referred to as puff or puff action below.

With the aim of further improving the quality of user experience at the time of using such an inhaler device, various technologies are under development. For example, PTL 1 describes a technology to radiate light, detect the phosphorescent characteristics of reflected light, and control the operation of an inhaler device in accordance with a detection result.

### Citation List

### Patent Literature

Patent Literature 1: JP 2019-528710 A

### Summary of Invention

### Technical Problem

As described in PTL 1, temperature control at the time of heating a substrate can contribute to improvement in flavor. In addition, cleaning an inhaler device also contributes to improvement in flavor. However, PTL 1 does not refer to cleaning of an inhaler device.

The present disclosure is contemplated in view of the above problem, and it is an object of the present disclosure to provide a mechanism of making it possible to further improve the quality of user experience.

### Solution to Problem

To solve the above problem, one aspect of the present disclosure provides an aerosol generating system. The aerosol generating system includes a container having an internal space and an opening that communicates the internal space with an outside, at least one first detector that radiates light to the internal space and detects reflected light received, and a controller that determines whether an article inserted in the container is a substrate including an aerosol source in accordance with intensities of multiple rays of reflected light, detected by the at least one detector.

The aerosol generating system may further include a heater that heats the substrate accommodated in the container, and the controller may control an operation of the heater in accordance with a determination result as to whether an article inserted in the container is the substrate.

When the controller determines that an article inserted in the container is the substrate, the controller may cause the heater to start performing heating.

When the controller determines that an article inserted in the container is the substrate, the controller may permit the heater to perform heating, and, when the controller determines that an article inserted in the container is not the substrate, the controller may prohibit the heater from performing heating.

The aerosol generating system may include the two detectors, and the two detectors may be disposed at locations different from each other.

The controller may cause each of the two detectors to operate at timing different from each other.

The controller may cause one of the two detectors to operate and the other one of the two detectors to sleep.

The two detectors may be disposed at locations different from each other in an insertion direction of the substrate.

The two detectors may be disposed at locations that at least partially overlap each other in an insertion direction of the substrate.

The two detectors may be disposed apart from each other by a diameter or greater of a thickest part of an article other than the substrate, estimated to be inserted in the container.

The two detectors may be disposed at locations at which an angle formed by directions to radiate light is larger than or equal to 90 degrees and smaller than or equal to 270 degrees in a plane orthogonal to an insertion direction of the substrate.

The two detectors may be disposed at locations at which an angle formed by directions to radiate light is 180 degrees in a plane orthogonal to the insertion direction of the substrate.

The controller may determine whether an article inserted in the container is the substrate in accordance with intensities of multiple rays of reflected light, detected at different timings by the same detector.

The aerosol generating system may further include at least any one of the substrate and an article other than the substrate, estimated to be inserted in the container.

To solve the above problem, another aspect of the present disclosure provides an information processing method that is executed by a computer. The information processing method includes determining whether an article inserted in a container having an internal space and an opening that communicates the internal space with an outside is a substrate including an aerosol source in accordance with intensities of multiple rays of reflected light, detected by at least one detector that radiates light to the internal space of the container and detects reflected light received.

### Advantageous Effects of Invention

According to the present disclosure as described above, a mechanism of making it possible to further improve the quality of user experience.

### Brief Description of Drawings

[Fig. 1] Fig. 1 is a schematic diagram that schematically illustrates an internal configuration example of an inhaler device.
[Fig. 2] Fig. 2 is an overall perspective view of the inhaler device according to an embodiment.
[Fig. 3] Fig. 3 is an overall perspective view of the inhaler device according to the present embodiment in a state where a stick substrate is held.
[Fig. 4] Fig. 4 is a diagram that schematically illustrates a configuration around a container of the inhaler device according to the present embodiment.
[Fig. 5] Fig. 5 is a schematic diagram that illustrates a configuration around an optical sensor of the inhaler device according to the present embodiment in detail.
[Fig. 6] Fig. 6 is a schematic diagram of the container of the inhaler device according to the present embodiment when viewed from an opening side (that is, upper side).
[Fig. 7] Fig. 7 is a block diagram that illustrates a configuration of the optical sensor in the inhaler device according to the present embodiment.
[Fig. 8] Fig. 8 is a timing chart that illustrates an example of the operation of the optical sensor on a time axis.
[Fig. 9] Fig. 9 is a view that illustrates an example of the configuration of a swab according to the present embodiment.
[Fig. 10] Fig. 10 is a diagram that schematically illustrates a state where the container in which a stick substrate is inserted is viewed from the opening side (that is, upper side).
[Fig. 11] Fig. 11 is a diagram that schematically illustrates a state where the container in which a swab is inserted is viewed from an opening side (that is, upper side).
[Fig. 12] Fig. 12 is a flowchart that illustrates an example of the flow of a process that is executed by the inhaler device according to the present embodiment.

### Description of Embodiments

Hereinafter, a preferred embodiment of the present disclosure will be described in detail with reference to the attached drawings. In the specification and the drawings, like reference signs denote structural elements having substantially the same functional components, and the description will not be repeated.

In the specification and the drawings, elements each having substantially the same functional configuration can be distinguished from one another by suffixing different alphabets to the same reference signs. For example, a plurality of elements each having substantially the same functional configuration is distinguished from one another like an optical sensor 170A and an optical sensor 170B where necessary. However, when a plurality of elements each having substantially the same functional configuration does not need to be distinguished from one another, only the same reference sign is assigned. When, for example, the optical sensor 170A and the optical sensor 170B do not need to be distinguished from each other, the optical sensors 170A, 170B are simply referred to as optical sensors 170.

### 1. Configuration example of inhaler device

### (1) Internal configuration example

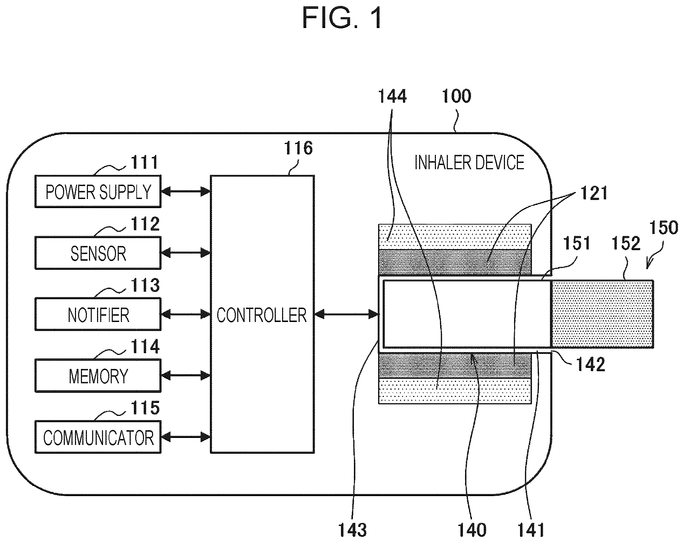

Fig. 1 is a schematic diagram that schematically illustrates an internal configuration example of an inhaler device. As illustrated in Fig. 1, an inhaler device 100 according to the present configuration example includes a power supply 111, a sensor 112, a notifier 113, a memory 114, a communicator 115, a controller 116, a heater 121, a container 140, and a heat insulator 144.

The power supply 111 stores electric power. The power supply 111 supplies electric power to the structural elements of the inhaler device 100 under the control of the controller 116. The power supply 111 can be a rechargeable battery, such as a lithium ion secondary battery.

The sensor 112 acquires various items of information regarding the inhaler device 100. In an example, the sensor 112 may be a pressure sensor such as a capacitor microphone, a flow sensor, or a temperature sensor, and acquire a value generated in accordance with the user's inhalation. In another example, the sensor 112 is an input device that receives information input by the user, such as a button and a switch.

The notifier 113 notifies the user of information. The notifier 113 may be a light-emitting device that emits light, a display device that displays an image, a sound output device that outputs sound, or a vibration device that vibrates.

The memory 114 stores various pieces of information for the operation of the inhaler device 100. The memory 114 is, for example, a non-volatile storage medium, such as a flash memory.

The communicator 115 is a communication interface capable of communication in conformity with any wired or wireless communication standard. For example, a standard using Wi-Fi (registered trademark), Bluetooth (registered trademark), Bluetooth Low Energy (BLE) (registered trademark), near field communication (NFC), or a low-power wide area (LPWA) can be adopted as such a communication standard.

The controller 116 functions as an arithmetic processing unit and a control device and controls the overall operations in the inhaler device 100 in accordance with various programs. The controller 116 is implemented by, for example, an electronic circuit, such as a central processing unit (CPU) and a microprocessor.

The container 140 has an internal space 141. The container 140 holds the stick substrate 150 while accommodating part of the stick substrate 150 in the internal space 141. The container 140 has the opening 142 that allows the internal space 141 to communicate with outside. The container 140 accommodates the stick substrate 150 that is inserted into the internal space 141 through the opening 142. For example, the container 140 is a tubular body having the opening 142 and a bottom 143 at its ends, and defines the pillar-shaped internal space 141. An airflow path for supplying air to the internal space 141 is connected to the container 140. An air inlet hole that is an inlet for air into the airflow path is disposed at, for example, the side of the inhaler device 100. An air outlet hole that is an outlet for air from the airflow path to the internal space 141 is disposed at, for example, the bottom 143.

The stick substrate 150 includes a substrate 151 and an inhalation port 152. The substrate 151 includes an aerosol source. The aerosol source includes a flavor component that is either derived from tobacco or not derived from tobacco. For the inhaler device 100 that is a medical inhaler such as a nebulizer, the aerosol source may include a medicine. The aerosol source may be, for example, a liquid, such as polyhydric alcohol and water, containing a flavor component derived from tobacco or not derived from tobacco. Examples of the polyhydric alcohol include glycerin and propylene glycol. The aerosol source may also be a solid containing a flavor component derived from tobacco or not derived from tobacco. The stick substrate 150 held by the container 140 includes the substrate 151 at least partially accommodated in the internal space 141 and the inhalation port 152 at least partially protruding from the opening 142. When a user inhales with the inhalation port 152 protruding from the opening 142 in his/her mouth, air flows into the internal space 141 through the airflow path (not illustrated), and the air and an aerosol generated from the substrate 151 reach the inside of the mouth of the user.

The heater 121 heats the aerosol source to atomize the aerosol source and generate the aerosol. In the example illustrated in Fig. 1, the heater 121 has a film-like shape and surrounds the outer circumference of the container 140. Subsequently, heat produced from the heater 121 heats the substrate 151 of the stick substrate 150 from the outer circumference, and an aerosol is generated. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed such that the heater 121 protrudes from the bottom 143 of the container 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed such that the heater 121 covers the bottom 143 of the container 140. In still another example, the heater 121 may be implemented as a combination of two or more selected from a first heater that covers the outer circumference of the container 140, a second heater having the blade-like shape, and a third heater that covers the bottom 143 of the container 140.

In another example, the container 140 may include an opening/closing mechanism that at least partially opens and closes an outer shell defining the internal space 141. Examples of the opening/closing mechanism include a hinge. In addition, the container 140 may accommodate the stick substrate 150 inserted into the internal space 141 while sandwiching the stick substrate 150, by opening and closing the outer shell. In this case, the heater 121 may be at the sandwiching position of the container 140 and may produce heat while pressing the stick substrate 150.

In addition, means for atomizing the aerosol source is not limited to heating by the heater 121. For example, the means for atomizing the aerosol source may be induction heating.

The inhaler device 100 and the stick substrate 150 may be regarded as making up an aerosol generating system to cooperatively generate an aerosol. Alternatively, the inhaler device 100 may be regarded as including the stick substrate 150.

### (2) External configuration example

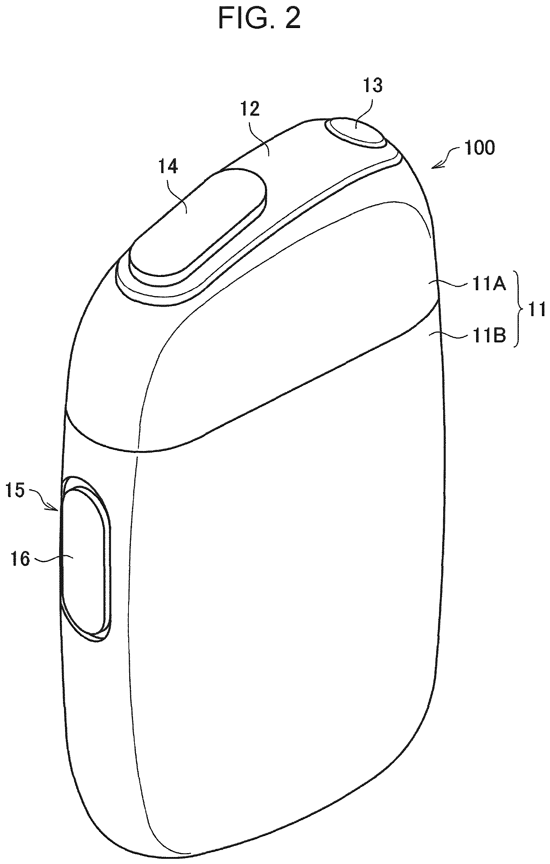

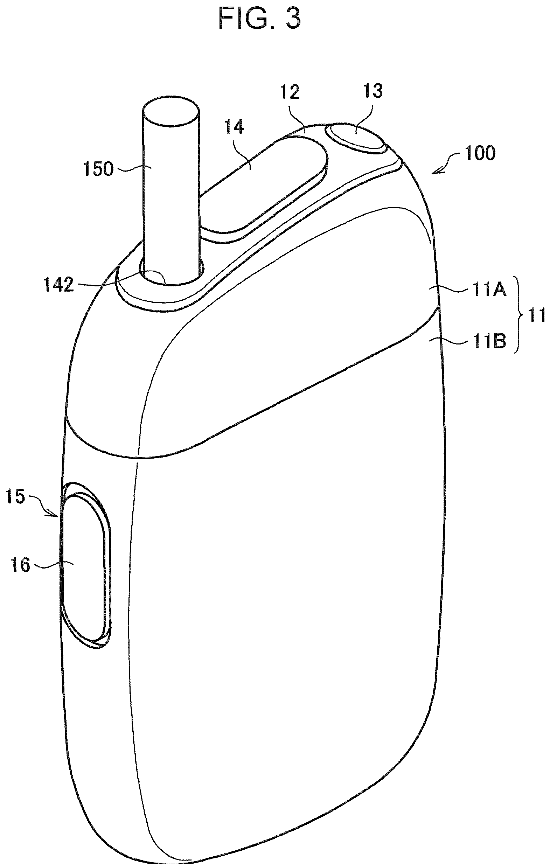

Fig. 2 is an overall perspective view of the inhaler device 100 according to an embodiment. Fig. 3 is an overall perspective view of the inhaler device 100 according to the present embodiment in a state where the stick substrate 150 is held.

As illustrated in Figs. 2 and 3, the inhaler device 100 includes a top housing 11A, a bottom housing 11B, a cover 12, a switch 13, a lid 14, a vent hole 15, and a cap 16. The top housing 11A and the bottom housing 11B are connected to each other to make up an outermost outer housing 11 of the inhaler device 100. The outer housing 11 has such a size as to fit in user's hand. When the user uses the inhaler device 100, the user is able to hold the inhaler device 100 by hand and inhale a flavor.

The top housing 11A has an opening (not illustrated). The cover 12 is coupled to the top housing 11A so as to close the opening. As shown in Fig. 3, the cover 12 has an opening 142 through which the stick substrate 150 can be inserted. The lid 14 is configured to open and close the opening 142 of the cover 12.

The switch 13 is used to switch the activation of the inhaler device 100 between an on state and an off state. For example, when the user operates the switch 13 in a state where the stick substrate 150 is inserted in the internal space 141 through the opening 142 as illustrated in Fig. 3, electric power is supplied from the power supply 111 to the heater 121, and the stick substrate 150 can be heated without burning the stick substrate 150. Once the stick substrate 150 is heated, an aerosol is generated from the aerosol source included in the stick substrate 150, and the flavor of the flavor source is taken into the aerosol. The user is able to inhale the aerosol containing a flavor by inhaling a part (a part illustrated in Fig. 3, that is, the inhalation port 152) of the stick substrate 150, protruding from the inhaler device 100.

The vent hole 15 is a vent hole for introducing air into the internal space 141. Air taken into the inhaler device 100 through the vent hole 15 is, for example, introduced into the internal space 141 from the bottom 143 of the container 140. The cap 16 is configured to be attachable to and detachable from the bottom housing 11B. When the cap 16 is attached to the bottom housing 11B, the vent hole 15 is formed between the bottom housing 11B and the cap 16. The cap 16 can have, for example, a through-hole, a cutout, or the like (not illustrated).

### 2. Technical features

### (1) Detailed configuration around container 140

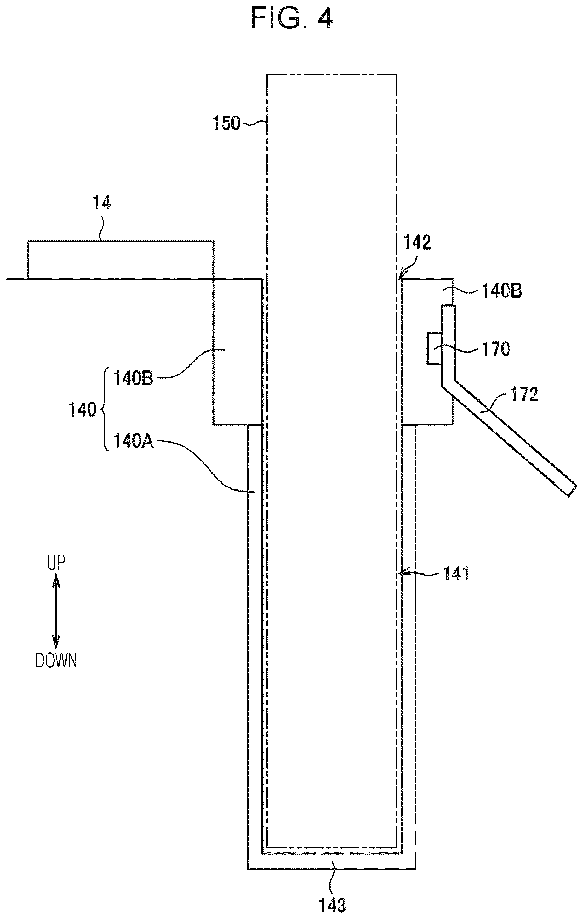

Fig. 4 is a diagram that schematically illustrates a configuration around the container 140 of the inhaler device 100 according to the present embodiment. Fig. 4 schematically illustrates a state where the stick substrate 150 is accommodated in the container 140. As illustrated in Fig. 4, the inhaler device 100 includes the lid 14, a stick lower part container 140A, a guide 140B, the opening 142, the bottom 143, an optical sensor 170, and a circuit board 172. A direction in which the stick substrate 150 is inserted to and removed from the inhaler device 100 is also referred to as up and down direction. Then, the insertion direction of the stick substrate 150 is referred to as lower side, and the removal direction of the stick substrate 150 is referred to as upper side.

The stick lower part container 140A is a closed-end tubular body that is a bottom 143-side part of the container 140. The stick lower part container 140A accommodates a bottom 143-side part of the stick substrate 150 inserted in the internal space 141 through the opening 142.

The guide 140B is an open-end tubular body that is an opening 142-side part of the container 140. The guide 140B accommodates a part not accommodated in the stick lower part container 140A, of the part accommodated in the container 140 in the stick substrate 150 inserted in the internal space 141 through the opening 142. Furthermore, the guide 140B functions as a guide for making it easy to insert the stick substrate 150 into the stick lower part container 140A. For example, the guide 140B may have a greater bore diameter than the stick lower part container 140A or may have such a funnel shape that the bore diameter gradually reduces from the upper side toward the lower side.

The optical sensor 170 radiates light to the internal space 141 and detects reflected light received. The optical sensor 170 is an example of the detector according to the present embodiment and is included in the sensor 112. The optical sensor 170 is, for example, an integrated circuit (IC) equipped with an infrared proximity sensor. In this case, the optical sensor 170 radiates infrared rays to the internal space 141 and detects infrared rays reflected from an object to be detected, such as an object accommodated in the internal space 141 and an inner wall of the container 140.

The optical sensor 170 is disposed at a place where the optical sensor 170 can radiate light to the internal space 141. For example, the optical sensor 170 is disposed at the guide 140B. Specifically, the optical sensor 170 is embedded in the guide 140B. The optical sensor 170 detects light reflected from an object to be detected, such as an object accommodated in the internal space 141 and the inner wall of the guide 140B.

Here, the heater 121 is disposed so as to surround the outer circumference of the stick lower part container 140A. On the other hand, the heater 121 is not disposed around the outer circumference of the guide 140B. The guide 140B may be made of a material having a lower thermal conductivity than the material of the stick lower part container 140A. For this reason, the optical sensor 170 can perform detection with light without the influence of heating of the stick substrate 150.

The inner wall of the guide 140B may be black. When the inner wall of the guide 140B is black, it is possible to suppress reflection of light radiated from the optical sensor 170. When the fact that the stick substrate 150 can be a color, such as white, that relatively easily reflects light is taken into consideration, it is possible to significantly vary the intensity of reflected light between a case where the stick substrate 150 is inserted and another case.

The circuit board 172 is a board on which the optical sensor 170 is mounted. The circuit board 172 is, for example, flexible printed circuits (FPC) circuit. The circuit board 172 is connected to the controller 116 by, for example, a connector or solder.

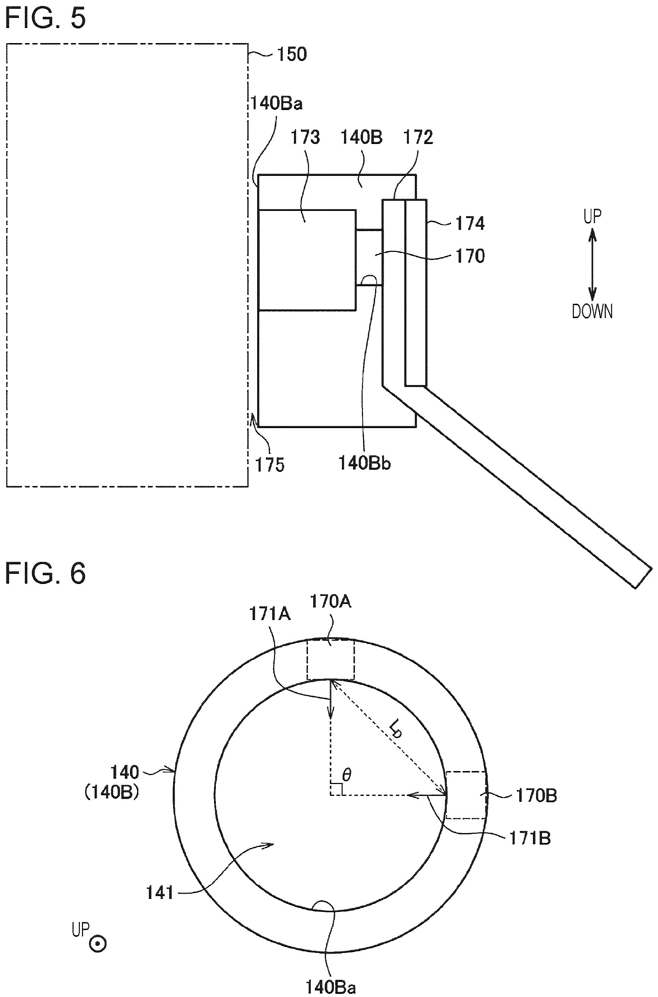

Fig. 5 is a schematic diagram that illustrates the configuration of the inhaler device 100 according to the present embodiment around the optical sensor 170 in detail. As illustrated in Fig. 5, the inhaler device 100 further includes a light transmission filter 173 and a reinforcing plate 174.

The light transmission filter 173 is a filter that transmits light radiated from the optical sensor 170. The light transmission filter 173 is, for example, an infrared transmission filter in a case where the optical sensor 170 is an infrared proximity sensor. The material of the light transmission filter 173 is not limited. The material of the light transmission filter 173 may be resin or glass or may be formed by applying a light transmission coating to a transparent resin. The light transmission filter 173 may be colored. When the light transmission filter 173 is colored, the optical sensor 170 can be hidden in appearance. A hole 140Bb is provided in an inner wall 140Ba of the guide 140B, and the optical sensor 170 is disposed so as to be embedded in the hole 140Bb. The light transmission filter 173 is disposed so as to close the hole 140Bb and forms the inner wall 140Ba of the guide 140B. With the above configuration, it is possible to smooth the inner wall 140Ba of the guide 140B. With the light transmission filter 173, it is possible to maintain airtightness so that sidestream smoke or the like flowing in from outside the stick does not touch the optical sensor 170.

The clearance 175 is a gap provided between the stick substrate 150 accommodated in the container 140 and the inner wall 140Ba of the guide 140B. The clearance 175 may be provided such that the distance between the stick substrate 150 and the inner wall 140Ba of the guide 140B ranges from 1 mm to 2 mm.

The reinforcing plate 174 is a plate-shaped member having a predetermined rigidity. The reinforcing plate 174 is disposed so as to cover the back side of the circuit board 172 on which the optical sensor 170 is disposed on the front side and reinforces the optical sensor 170 and the circuit board 172.

Fig. 6 is a schematic diagram of the container 140 of the inhaler device 100 according to the present embodiment when viewed from the opening 142 side (that is, upper side). As illustrated in Fig. 6, the inhaler device 100 may include the two optical sensors 170 (170A, 170B). The optical sensor 170A and the optical sensor 170B are disposed apart from each other, and the distance therebetween is L_{D}. Then, a direction 171A in which the optical sensor 170A radiates light (hereinafter, also referred to as radiation direction 171A) and a direction 171B in which the optical sensor 170B radiates light (hereinafter, also referred to as radiation direction 171B) form an angle θ in a plane orthogonal to the up and down direction. When the inhaler device 100 includes the plurality of optical sensors 170 and additionally an appropriate distance L_{D} and an appropriate angle θ are provided, it is possible to further accurately perform a determination on an article inserted in the container 140. A determination process using the optical sensors 170 will be described in detail later.

### (2) Configuration of optical sensor 170

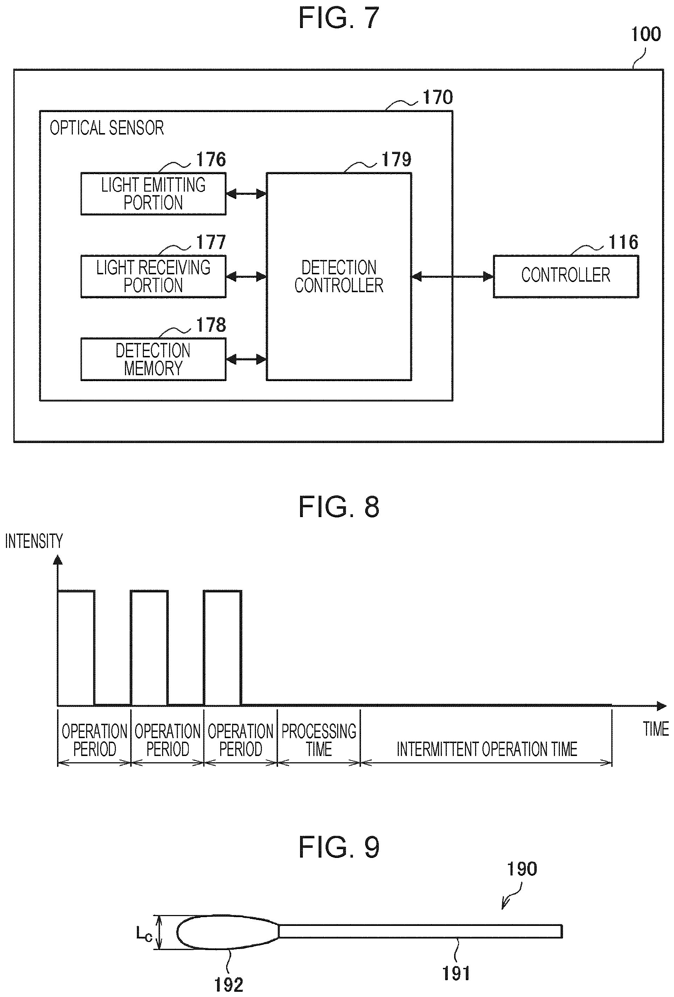

Next, the configuration of the optical sensor 170 will be described in detail with reference to Fig. 7. Fig. 7 is a block diagram that illustrates the configuration of the optical sensor 170 in the inhaler device 100 according to the present embodiment.

As illustrated in Fig. 7, the optical sensor 170 includes a light emitting portion 176, a light receiving portion 177, a detection memory 178, and a detection controller 179. Then, the optical sensor 170 is connected to the controller 116. The optical sensor 170 operates under control of the controller 116.

The light emitting portion 176 radiates light to the internal space 141. The light emitting portion 176 is made up of a light-emitting element, such as a laser diode (LD) and a light emitting diode (LED). In the present embodiment, the light emitting portion 176 is an infrared LD and radiates infrared rays. The light receiving portion 177 detects reflected light of light radiated from the light emitting portion 176. Infrared rays radiated by the light emitting portion 176 may be vertical cavity surface emitting laser (VCSEL). The operation of the light emitting portion 176 will be described in detail with reference to Fig. 8.

Fig. 8 is a timing chart that illustrates an example of the operation of the optical sensor 170 on a time axis. The abscissa axis of Fig. 8 represents time, and the time flows from the left-hand side to the right-hand side. The ordinate axis of Fig. 8 represents the intensity of light radiated from the light emitting portion 176. As illustrated in Fig. 8, the light emitting portion 176 performs pulsed light emission at a predetermined period. The period is also referred to as operation period. The light emitting portion 176 repeats pulsed light emission three times and then stops light emission in a processing time and an intermittent operation time. The processing time is a time during which a process based on reflected light detected by the light receiving portion 177 is executed. The intermittent operation time is a time to when the next pulsed light emission is performed. The light emitting portion 176 repeatedly performs a series of operations including pulsed light emission described with reference to Fig. 8 and a stop of emission.

The detection controller 179 controls the operation of each of the structural elements of the optical sensor 170. Hereinafter, an example of a process that is executed by the detection controller 179 will be described. These processes are basically executed in the processing time described with reference to Fig. 8.

In an example, the detection controller 179 calculates a value indicating the intensity of reflected light detected by the light receiving portion 177. The calculated value indicating the intensity of reflected light is also referred to as a detection value hereinafter. The detection controller 179 calculates a larger detection value as the detected intensity of reflected light increases. The relationship between the intensity of reflected light and detection value may be linear.

In another example, the detection controller 179 may calculate a distance to an object to be detected, having reflected light radiated from the optical sensor 170, that is, a distance between an object to be detected and the optical sensor 170, in accordance with a detection value. More specifically, the detection controller 179 calculates a shorter distance as the detection value increases, that is, as the intensity of reflected light increases. On the other hand, the detection controller 179 calculates a longer distance as the detection value reduces, that is, as the intensity of reflected light decreases.

In another example, the detection controller 179 controls the operation of the light emitting portion 176. More specifically, the detection controller 179 may control at least any one of the number of times of pulsed light emission, operation period, and intermittent operation time, illustrated in Fig. 8. The detection controller 179 may control the intensity of infrared rays radiated from the light emitting portion 176 by controlling a current value (hereinafter, also referred to as LD current value) applied to the light emitting portion 176.

In another example, the detection controller 179 provides notification of information to the controller 116. For example, the detection controller 179 may notify the controller 116 of the calculated detection value. The detection controller 179 causes the detection memory 178 to store the calculated detection value. Then, when the detection value exceeds a predetermined threshold (hereinafter, also referred to as notified threshold), the detection controller 179 may notify the controller 116 of that fact. This notification is also referred to as interrupt notification hereinafter. In this case, the controller 116 reads the detection value stored in the detection memory 178 upon reception of an interrupt notification as a trigger. Such a process related to a detection value may be similarly executed for a distance to an object to be detected. In other words, the detection controller 179 may notify the controller 116 of the calculated distance. Alternatively, the detection controller 179 may store the calculated distance in the detection memory 178 and, when the calculated distance exceeds a notified threshold, notify the controller 116 of the fact.

The interrupt notification may be a notification that indicates that an article of some kind is inserted in the container 140. In this case, the controller 116 may execute a predetermined process upon reception of an interrupt notification as a trigger. An example of the predetermined process can include determining whether a stick determination condition (described later) is satisfied, heating control in accordance with a determination result, and the like. With the above configuration, since the predetermined process is executed only when an interrupt notification is received, it is possible to reduce processing load on the controller 116.

In another example, the detection controller 179 may perform calibration. Specifically, the detection controller 179 may adjust the relationship between the intensity of reflected light detected by the light receiving portion 177 and a detection value to be calculated such that the same detection value is calculated under a predetermined condition. By performing calibration, it is possible to exclude a deviation of detection value due to temperature, vibration, or the like, or exclude the influence of aged degradation or the like of the light emitting portion 176 or the light receiving portion 177.

Fig. 8 illustrates an example in which the number of times of pulsed light emission by the light emitting portion 176 is three; however, the number of times of pulsed light emission is not limited. When the number of times of pulsed light emission by the light emitting portion 176 is multiple, the detection controller 179 may execute a process using a detection result, may execute a process using a multiple number of detection results by the light receiving portion 177, or may execute a process using one or some of detection results among a multiple number of detection results by the light receiving portion 177.

The detection memory 178 stores a program that the detection controller 179 executes, various pieces of data, and the like. The detection memory 178 is an example of the memory according to the present embodiment. The detection memory 178 is implemented by, for example, a register. The detection memory 178 stores various setting values, that is, the operation period of pulsed light emission of infrared rays, the intermittent operation time, the notified threshold, the LD current value, and the like, used at the time of control by the detection controller 179.

The controller 116 and the detection controller 179 communicate with each other. The controller 116 and the detection controller 179 communicate with each other by, for example, a serial communication interface, such as inter-integrated circuit (I2C). The controller 116 controls the operations of the structural elements of the optical sensor 170 via the detection controller 179.

The controller 116 controls to switch the mode of the optical sensor 170 to an operation mode to detect reflected light or to a sleep mode to stop detection of reflected light. Specifically, in the sleep mode, the controller 116 controls the light emitting portion 176 such that the light emitting portion 176 stops radiation of light and controls the light receiving portion 177 such that the light receiving portion 177 stops detection of reflected light. In the operation mode, the controller 116 controls the light emitting portion 176 such that the light emitting portion 176 radiates light and controls the light receiving portion 177 such that the light receiving portion 177 detects reflected light. When the controller 116 controls switching of the mode of the optical sensor 170, it is possible to reduce power consumption as compared to a case where detection of reflected light with the optical sensor 170 is constantly performed.

The controller 116 causes the detection memory 178 to store various setting values used at the time of control by the detection controller 179. The controller 116 receives various pieces of information, such as an interrupt notification, from the detection controller 179 or reads information stored in the detection memory 178.

Here, the detection memory 178 may be made up of a volatile storage medium or may be made up of a non-volatile storage medium. In a case where the detection memory 178 is made up of a non-volatile storage medium, when electric power is supplied again after electric power supplied to the optical sensor 170 is interrupted, various setting values stored in the detection memory 178 are initialized. When various setting values are initialized, the controller 116 may store various setting values before initialization in the detection memory 178 again.

Instead of the sleep mode, the controller 116 may control the mode to a power off mode in which supply of electric power to the optical sensor 170 is stopped. In a case where the detection memory 178 is made up of a volatile storage medium and such control is executed, the controller 116 causes the detection memory 178 to store various setting values before initialization again at the time of switching the mode of the optical sensor 170 from the power off mode to the operation mode. In the sleep mode, the controller 116 may control to maintain supply of electric power to the detection memory 178 of the optical sensor 170. Thus, in a case where the detection memory 178 is made up of a volatile storage medium, it is not necessary to store various setting values before initialization in the detection memory 178 each time the mode is switched from the sleep mode to the operation mode. In the sleep mode, the controller 116 may control to maintain supply of electric power to only the memory of part of the detection memory 178 of the optical sensor 170.

### (3) Determination of inserted article

A crud, such as soil and foreign matter, can remain in the internal space 141. In an example, a content cannot overflow from the distal end of the heated stick substrate 150 and can remain in the internal space 141 as a crud. In a state where a crud remains, it is difficult to appropriately heat the stick substrate 150, with the result that it is difficult to provide the user with good flavor. For this reason, the container 140 is preferably periodically cleaned. When the crud is removed by cleaning, it is possible to appropriately heat the stick substrate 150, with the result that it is possible to provide a good flavor to the user. An example of a cleaning article used to clean the container 140 will be described with reference to Fig. 9.

Fig. 9 is a view that illustrates an example of the configuration of a swab 190 according to the present embodiment. As illustrated in Fig. 9, the swab 190 includes a shaft 191 and a fiber mass 192.

The shaft 191 is a member formed in a longitudinal shape. For example, the shaft 191 is formed by rolling paper sheet.

The fiber mass 192 is formed by winding fiber at one end of the shaft 191 and bonding the fiber. A selected shape, such as a teardrop shape, a cylindrical shape, a spherical shape, and a shape having random unevenness, can be adopted as the shape of the fiber mass 192. Examples of the fiber that is a component of the fiber mass 192 include various natural fibers (cotton, silk, wool, or the like), regenerated fibers (rayon, cupro, or the like), and synthetic fibers (polyester fiber, polypropylene fiber, or the like). The fiber mass 192 may contain liquid, such as alcohol. The fiber mass 192 may be disposed at one end of the shaft 191 as shown in Fig. 9 or may be disposed at each end of the shaft 191.

The swab 190 is an example of the cleaning article. The user holds the shaft 191 and inserts the fiber mass 192 into the internal space 141 through the opening 142. Then, the user moves the fiber mass 192 so that the fiber mass 192 is rubbed against the container 140. As a result, a crud remaining in the container 140 adheres to the fiber mass 192 and is removed. In this way, the container 140 is cleaned.

The swab 190 is configured to be thinner than the stick substrate 150. Particularly, a diameter (more specifically, a diameter of the fiber mass 192 at the thickest part) L_{C} of the swab 190 is configured to be less than a diameter (more specifically, a diameter at the thinnest part) L_{S} of the stick substrate 150. In an example, the diameter L_{C} of the swab 190 may be less than or equal to a half of the diameter L_{S} of the stick substrate 150 and preferably less than or equal to a quarter of the diameter L_{S}. With the above configuration, when the swab 190 is inserted in the container 140, a large gap is ensured between the inner wall 140Ba of the guide 140B and the swab 190. As a result, the fiber mass 192 can be freely moved in the internal space 141, so it is possible to improve cleaning efficiency.

The swab 190 is an example of the article other than the stick substrate 150, estimated to be inserted in the container 140. The inhaler device 100 and the swab 190 may be regarded as making up the aerosol generating system. Alternatively, the inhaler device 100 may be regarded as including the swab 190.

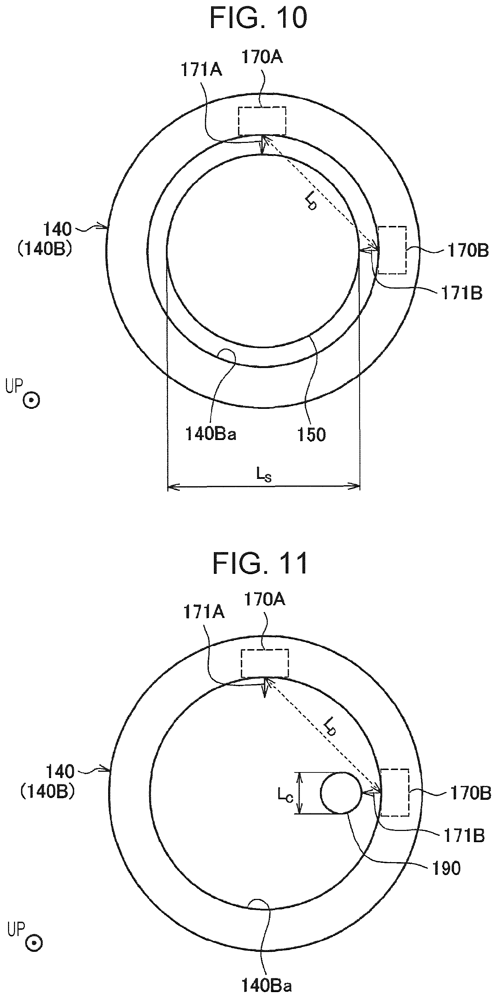

A difference between the diameter L_{C} of the swab 190 and the diameter L_{S} of the stick substrate 150 can also be used to identify an article inserted in the container 140 (hereinafter, also referred to as inserted article). Detection values to be detected by the optical sensor 170A and the optical sensor 170B significantly vary between a case where the inserted article is the stick substrate 150 and a case where the inserted article is the swab 190. This point will be described with reference to Figs. 10 and 11.

Fig. 10 is a diagram that schematically illustrates a state where the container 140 in which the stick substrate 150 is inserted is viewed from the opening 142 side (that is, upper side). As illustrated in Fig. 10, the diameter L_{S} of the stick substrate 150 is longer than the distance L_{D} between the optical sensor 170A and the optical sensor 170B. As the clearance 175 has been described with reference to Fig. 5, the distance between the stick substrate 150 and the inner wall 140Ba of the guide 140B ranges from about 1 mm to about 2 mm. For this reason, as shown in Fig. 10, once the stick substrate 150 is inserted in the container 140, any part of the inner wall 140Ba of the guide 140B is located extremely close proximity to the stick substrate 150. As a result, light radiated from any of the optical sensor 170A and the optical sensor 170B is also reflected by the stick substrate 150 located in extremely close proximity. Therefore, a detection value detected by the optical sensor 170A and a detection value detected by the optical sensor 170B are large and mutually equivalent values.

Fig. 11 is a diagram that schematically illustrates a state where the container 140 in which the swab 190 is inserted is viewed from the opening 142 side (that is, upper side). As illustrated in Fig. 11, the diameter L_{C} of the swab 190 is remarkably shorter than the distance L_{D} between the optical sensor 170A and the optical sensor 170B. For this reason, as illustrated in Fig. 11, once the swab 190 is inserted in the container 140, the distance between the inner wall 140Ba of the guide 140B and the swab 190 significantly varies depending on the location of the inner wall 140Ba. As a result, a detection value of at least one of the optical sensor 170A and the optical sensor 170B is remarkably less than that in a case where the stick substrate 150 is inserted in the container 140. This is because the location of at least one of the optical sensor 170A and the optical sensor 170B is a location far from the swab 190 or where radiated light is not reflected by the swab 190. In the example illustrated in Fig. 11, the detection value of the optical sensor 170B has an equivalent magnitude to that in a case where the stick substrate 150 is inserted, while the detection value of the optical sensor 170A remarkably reduces.

Then, the controller 116 according to the present embodiment determines whether the inserted article is the stick substrate 150 in accordance with detection values detected by the two optical sensors 170. In an example, the controller 116 determines that the inserted article is the stick substrate 150 when the stick determination condition is satisfied. An example of the stick determination condition is that both the detection value of the optical sensor 170A and the detection value of the optical sensor 170B are greater than or equal to a predetermined threshold (hereinafter, also referred to as stick determination threshold). On the other hand, the controller 116 determines that the inserted article is not the stick substrate 150 when the stick determination condition is not satisfied. In other words, the controller 116 determines that the inserted article is not the stick substrate 150 when at least any one of the detection value of the optical sensor 170A and the detection value of the optical sensor 170B is less than the stick determination threshold. The controller 116 may determine that the inserted article is the swab 190 when the stick determination condition is not satisfied. The stick determination threshold just needs to be set to a selected value at which the stick determination condition is satisfied when the inserted article is the stick substrate 150 and the stick determination condition is not satisfied when the inserted article is the swab 190. However, the diameter of the stick substrate 150 can vary depending on a brand or a production lot or the stick substrate 150 can have a distorted shape. For this reason, the stick determination threshold is desirably set to a value with a margin (that is, a relatively low value). The stick determination threshold may be the same value as the above-described notified threshold. With the above configuration, it is possible to determine whether the inserted article is the stick substrate 150.

Here, a positional relationship between the two optical sensors 170 will be described with reference to Figs. 6 and 11.

As illustrated in Fig. 6, the optical sensor 170A and the optical sensor 170B are disposed at locations different from each other. As a result, the optical sensor 170A and the optical sensor 170B are capable of radiating light from different locations at different angles to the inserted article. With the above configuration, it is possible not to satisfy the stick determination condition when the inserted article is the swab 190. In other words, it is possible to improve the accuracy of determination as to whether the inserted article is the stick substrate 150.

As illustrated in Fig. 11, the optical sensor 170A and the optical sensor 170B are desirably disposed apart from each other by a distance greater than or equal to the diameter L_{C} of the thickest part of the swab 190 in a plane orthogonal to the up and down direction. In other words, the distance L_{D} between the optical sensor 170A and the optical sensor 170B is desirably longer than the diameter L_{C} of the fiber mass 192. With the above configuration, when the swab 190 is inserted in the container 140, it is possible not to have the swab 190 present in the radiation direction 171A or the radiation direction 171B. As a result, it is possible not to satisfy the stick determination condition when the inserted article is the swab 190. In other words, it is possible to improve the accuracy of determination as to whether the inserted article is the stick substrate 150.

As illustrated in Fig. 6, the optical sensor 170A and the optical sensor 170B are desirably disposed at locations at which an angle θ formed by the directions 171A, 171B in which respective rays of light are radiated is larger than or equal to 90 degrees and smaller than or equal to 270 degrees in a plane orthogonal to the up and down direction. With the above configuration, it is possible to ensure the long distance L_{D} between the optical sensor 170A and the optical sensor 170B. As a result, it is possible not to satisfy the stick determination condition when the inserted article is the swab 190. In other words, it is possible to improve the accuracy of determination as to whether the inserted article is the stick substrate 150.

However, the optical sensor 170A and the optical sensor 170B are desirably disposed at locations at which an angle θ formed by the directions 171A, 171B in which respective rays of light are radiated is other than 180 degrees in a plane orthogonal to the up and down direction. With the above configuration, it is possible to prevent occurrence of crosstalk. The crosstalk is a phenomenon that light radiated from one of the optical sensor 170A and the optical sensor 170B is erroneously detected by the other. When the occurrence of crosstalk is prevented, it is possible to prevent erroneous determination as to whether the inserted article is the stick substrate 150.

The controller 116 may cause each of the optical sensor 170A and the optical sensor 170B to operate at timing different from each other. For example, the controller 116 may cause the optical sensor 170A and the optical sensor 170B to operate alternately. More specifically, in the example described with reference to Fig. 8, the controller 116 may cause the optical sensor 170A and the optical sensor 170B to alternately perform pulsed light emission three times each. With the above configuration, it is possible to further reliably prevent occurrence of crosstalk.

The controller 116 may cause one of the optical sensor 170A and the optical sensor 170B to operate and cause the other one of the optical sensor 170A and the optical sensor 170B to sleep. Causing the optical sensor 170 to operate means causing the optical sensor 170 to detect reflected light. Causing the optical sensor 170 to sleep means causing the optical sensor 170 to stop detection of reflected light. In other words, the controller 116 may set the mode of one of the optical sensor 170A and the optical sensor 170B to the operation mode and set the mode of the other one of the optical sensor 170A and the optical sensor 170B to the sleep mode or the power off mode. Then, the controller 116 may interrupt the sleep of the other one of the optical sensor 170A and the optical sensor 170B and start the other one only when the detection value of one of the optical sensor 170A and the optical sensor 170B in operation becomes greater than or equal to the stick determination threshold. In this case, it is possible to determine whether the inserted article is the stick substrate 150 by operating both the optical sensor 170A and the optical sensor 170B only when an article of some kind is inserted in the container 140. For this reason, it is possible to suppress power consumption. While one of the optical sensor 170A and the optical sensor 170B is in sleep, it is possible to reliably prevent occurrence of crosstalk.

The optical sensor 170A and the optical sensor 170B may be disposed at locations at which an angle θ formed by the directions 171A, 171B in which respective rays of light are radiated is 180 degrees in a plane orthogonal to the up and down direction. Even in this case, when the above-described crosstalk preventative measures are taken, it is possible to suppress crosstalk. Furthermore, with the above configuration, it is possible to set the longest distance L_{D} between the optical sensor 170A and the optical sensor 170B. As a result, it is possible not to satisfy the stick determination condition when the inserted article is the swab 190. In other words, it is possible to improve the accuracy of determination as to whether the inserted article is the stick substrate 150.

### (4) Heating control according to determination result of inserted article

The controller 116 may control the operation of the heater 121 in accordance with a determination result as to whether the inserted article is the stick substrate 150. More specifically, the controller 116 varies the operation of the heater 121 between when the inserted article is the stick substrate 150 and otherwise. With the above configuration, it is possible to further improve usability.

In an example, when the controller 116 determines that the inserted article is the stick substrate 150, the controller 116 may cause the heater 121 to start performing heating. On the other hand, when the controller 116 determines that the inserted article is not the stick substrate 150, the controller 116 does not cause the heater 121 to start performing heating. In other words, the controller 116 may automatically start heating only when the stick substrate 150 is inserted. With the above configuration, even when user operation, such as button depression, to provide instructions to start heating is not performed separately, heating is automatically started only by inserting the stick substrate 150 to the container 140, so it is possible to improve usability.

In another example, when the controller 116 determines that the inserted article is the stick substrate 150, the controller 116 permits the heater 121 to perform heating and, when the controller 116 determines that the inserted article is not the stick substrate 150, the controller 116 may prohibit the heater 121 from performing heating. When heating is permitted, the inhaler device 100 starts heating in a case where user operation, such as button depression, to provide instructions to start heating is performed. On the other hand, when heating is prohibited, the inhaler device 100 does not start heating in a case where user operation, such as button depression, to provide instructions to start heating is performed. With the above configuration, heating is not started even in a case where button operation is erroneously performed during cleaning, so it is possible to improve the safety of the user.

### (5) Flow of Process

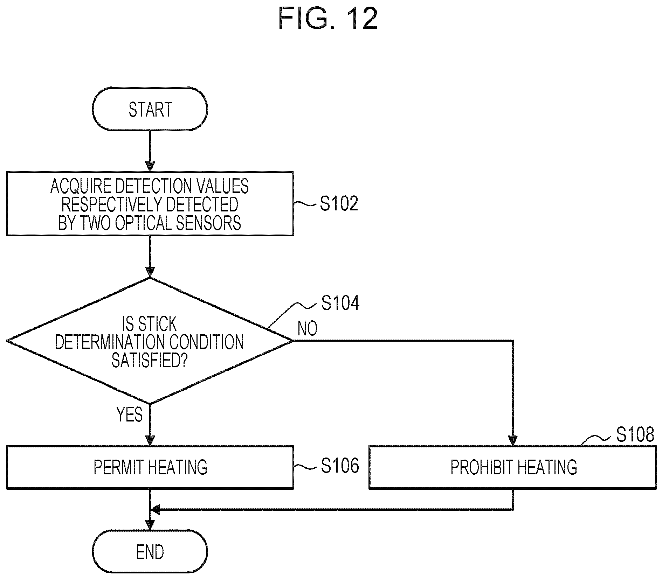

Fig. 12 is a flowchart that illustrates an example of the flow of a process that is executed by the inhaler device 100 according to the present embodiment.

As illustrated in Fig. 12, initially, the controller 116 acquires detection values respectively detected by the two optical sensors 170 (step S102). For example, the controller 116 reads the detection values stored in the detection memory 178 of the two optical sensors 170 upon reception of an interrupt notification from any one of the two optical sensors 170 as a trigger.

Subsequently, the controller 116 determines whether the stick determination condition is satisfied (step S104). More specifically, the controller 116 determines whether both the detection value of the optical sensor 170A and the detection value of the optical sensor 170B are greater than or equal to the stick determination threshold.

When it is determined that the stick determination condition is satisfied (YES in step S104), the controller 116 permits the heater 121 to perform heating (step S106).

When it is determined that the stick determination condition is not satisfied (NO in step S104), the controller 116 prohibits the heater 121 from performing heating (step S108).

### 3. Supplement

The preferred embodiment of the present disclosure has been described in detail with reference to the attached drawings; however, the present disclosure is not limited to those examples. It is obvious that persons having ordinary skill in the art in the field of technology to which the present disclosure belongs can conceive of various modifications or alterations within the scope of the technical idea recited in the claims, and these can also be naturally interpreted as belonging to the technical scope of the present disclosure.

In the above-described embodiment, an example in which the inhaler device 100 includes two optical sensors 170 has been described; however, the present disclosure is not limited to this example. The inhaler device 100 may include three or more optical sensors 170. In this case, at least two of the three or more optical sensors 170 just need to satisfy the above-described positional relationship.

In the above-described embodiment, an example in which it is determined whether the inserted article is the stick substrate 150 has been described; however, the present disclosure is not limited to this example. The controller 116 may determine the shape characteristics of the inserted article in accordance with detection values detected by the optical sensors 170. An example of the shape characteristics is the diameter of the inserted article. In other words, it may be determined whether the diameter of the inserted article is equivalent to the diameter of the stick substrate 150.

In the above-described embodiment, an example in which the stick determination condition relates to detection values detected by the optical sensors 170 has been described; however, the present disclosure is not limited to this example. The stick determination condition may relate to a distance to an object to be detected, calculated in accordance with detection values. For example, as a first condition, the stick determination condition may be that both a distance from the optical sensor 170A to an object to be detected and a distance from the optical sensor 170B to the object to be detected are less than a predetermined distance. The predetermined distance may be set as a distance corresponding to the stick determination threshold. With the above configuration as well, it is possible to determine whether the inserted article is the stick substrate 150.

In the above embodiment, an example in which the optical sensor 170A and the optical sensor 170B are disposed at different locations in a plane orthogonal to the up and down direction has been described; however, the present disclosure is not limited to this example.

Instead of or in addition to the configuration that the optical sensor 170A and the optical sensor 170 are disposed at different locations in a plane orthogonal to the up and down direction, the optical sensor 170A and the optical sensor 170 may be disposed at locations different from each other in the up and down direction. In an example, the optical sensor 170A may be disposed at the opening 142 side in the guide 140B, and the optical sensor 170B may be disposed at the bottom 143 side in the guide 140B. In a case where the optical sensor 170A and the optical sensor 170B are disposed at locations different from each other in the up and down direction, it is possible to have the radiation direction 171A and the radiation direction 171B not crossing over. Therefore, it is possible to further improve the accuracy of determination as to whether the inserted article is the stick substrate 150.

In a case where the optical sensor 170A and the optical sensor 170B are disposed at locations different from each other in the up and down direction, a change in the thickness of the inserted article in the up and down direction can be identified. For this reason, even when a part having a thickness equivalent to that of the stick substrate 150 is at part of a cleaning article in the up and down direction, it is possible to determine whether the inserted article is the stick substrate 150 or the cleaning article. However, it is assumed that the distance in the up and down direction between the optical sensor 170A and the optical sensor 170B is longer than the length in the up and down direction of the part of the cleaning article, having a thickness equivalent to that of the stick substrate 150.

Of course, the two optical sensors 170 may be disposed at locations that at least partially overlap each other in the up and down direction. To prevent transfer of heat from the heater 121 to the optical sensors 170, a range in which the optical sensors 170 can be disposed in the guide 140B is presumably limited. In terms of this point, with the above configuration, by, for example, disposing both the two optical sensors 170 at the opening 142 side relatively far from the heater 121, it is possible to prevent transfer of heat from the heater 121 to the optical sensors 170.

The controller 116 may determine whether the inserted article is the stick substrate 150 in accordance with a plurality of detection values detected at different timings by the same optical sensor 170. In an example, the controller 116 may determine that the inserted article is the stick substrate 150 when the range of a time-series change in detection value of the optical sensor 170 is smaller than a predetermined threshold. This is because, when the inserted article is the stick substrate 150, the stick substrate 150 does not significantly move in the container 140, so the range of a time-series change in detection value of the optical sensor 170 is estimated to be small. In another example, the controller 116 may determine that the inserted article is not the stick substrate 150 when the range of a time-series change in detection value of the optical sensor 170 is larger than or equal to a predetermined threshold. This is because, when the inserted article is the swab 190, the user significantly moves the swab 190 in the container 140 and, as a result, the detection value of the optical sensor 170 presumably significantly changes in time-series. With the above configuration, it is possible to improve the accuracy of determination as to whether the inserted article is the stick substrate 150.

When it is determined whether the inserted article is the stick substrate 150 in accordance with a time-series change in detection value, the number of the optical sensors 170 of the inhaler device 100 may be one. Here, a time-series change in detection value corresponds to a change in the thickness of the inserted article in the up and down direction. For this reason, even when a part having a thickness equivalent to that of the stick substrate 150 is at part of a cleaning article in the up and down direction, it is possible to determine whether the inserted article is the stick substrate 150 or the cleaning article in accordance with a time-series change in detection value.

A series of pieces of processing, executed by the devices, described in the specification, may be implemented by any one of software, hardware, and a combination of software and hardware. Programs that are components of software are prestored in, for example, recording media (more specifically, non-transitory computer-readable storage media) provided inside or outside the devices. The programs are, for example, loaded onto a RAM when a computer that controls the devices described in the specification runs the programs and are run on a processing circuit, such as a CPU. Examples of the storage media include a magnetic disk, an optical disk, a magneto-optical disk, and a flash memory. The computer programs may be distributed via, for example, a network, without using storage media. The computer may be an application specific integrated circuit (ASIC), a general-purpose processor that executes a function by loading a software program, a computer on a server used for cloud computing, or the like. A series of processes that are executed by the devices described in the specification may be processed by a plurality of computers in a distributed manner.

Pieces of processing described by using the flowchart and the sequence diagram in the specification may be not necessarily executed in order as illustrated. Some processing steps may be executed in parallel. An additional processing step may be adopted, and one or some of the processing steps may be omitted.

The following configurations also belong to the technical scope of the present disclosure.
(1) An aerosol generating system including:
   a container having an internal space and an opening that communicates the internal space with an outside;
   at least one first detector that radiates light to the internal space and detects reflected light received; and
   a controller that determines whether an article inserted in the container is a substrate including an aerosol source in accordance with intensities of multiple rays of reflected light, detected by the at least one detector.
(2) The aerosol generating system according to (1), wherein
   the aerosol generating system further includes a heater that heats the substrate accommodated in the container, and
   the controller controls an operation of the heater in accordance with a determination result as to whether an article inserted in the container is the substrate.
(3) The aerosol generating system according to (2), wherein, when the controller determines that an article inserted in the container is the substrate, the controller causes the heater to start performing heating.
(4) The aerosol generating system according to (2), wherein, when the controller determines that an article inserted in the container is the substrate, the controller permits the heater to perform heating, and, when the controller determines that an article inserted in the container is not the substrate, the controller prohibits the heater from performing heating.
(5) The aerosol generating system according to any one of (1) to (4), wherein
   the aerosol generating system includes the two detectors, and
   the two detectors are disposed at locations different from each other.
(6) The aerosol generating system according to (5), wherein the controller causes each of the two detectors to operate at timing different from each other.
(7) The aerosol generating system according to (5) or (6), wherein the controller causes one of the two detectors to operate and the other one of the two detectors to sleep.
(8) The aerosol generating system according to any one of (5) to (7), wherein the two detectors are disposed at locations different from each other in an insertion direction of the substrate.
(9) The aerosol generating system according to any one of (5) to (7), wherein the two detectors are disposed at locations that at least partially overlap each other in an insertion direction of the substrate.
(10) The aerosol generating system according to any one of (5) to (9), wherein the two detectors are disposed apart from each other by a diameter or greater of a thickest part of an article other than the substrate, estimated to be inserted in the container.
(11) The aerosol generating system according to (10), wherein the two detectors are disposed at locations at which an angle formed by directions to radiate light is larger than or equal to 90 degrees and smaller than or equal to 270 degrees in a plane orthogonal to an insertion direction of the substrate.
(12) The aerosol generating system according to (11), wherein the two detectors are disposed at locations at which an angle formed by directions to radiate light is 180 degrees in a plane orthogonal to the insertion direction of the substrate.
(13) The aerosol generating system according to any one of (1) to (12), wherein the controller determines whether an article inserted in the container is the substrate in accordance with intensities of multiple rays of reflected light, detected at different timings by the same detector.
(14) The aerosol generating system according to any one of (1) to (13), wherein the aerosol generating system further comprises at least any one of the substrate and an article other than the substrate, estimated to be inserted in the container.
(15) An information processing method that is executed by a computer, the information processing method including determining whether an article inserted in a container having an internal space and an opening that communicates the internal space with an outside is a substrate including an aerosol source in accordance with intensities of multiple rays of reflected light, detected by at least one detector that radiates light to the internal space of the container and detects reflected light received.

### Reference Signs List

- 100: inhaler device
- 111: power supply
- 112: sensor
- 113: notifier
- 114: memory
- 115: communicator
- 116: controller
- 121: heater
- 140: container
- 140A: stick lower part container
- 140B: guide
- 141: internal space
- 142: opening
- 143: bottom
- 144: heat insulator
- 150: stick substrate
- 170: optical sensor
- 172: circuit board
- 173: light transmission filter
- 174: reinforcing plate
- 175: clearance
- 176: light emitting portion
- 177: light receiving portion
- 178: detection memory
- 179: detection controller
- 190: swab
- 191: shaft
- 192: fiber mass

## Claims

1. An aerosol generating system comprising:
a container having an internal space and an opening that communicates the internal space with an outside;
at least one first detector that radiates light to the internal space and detects reflected light received; and
a controller that determines whether an article inserted in the container is a substrate including an aerosol source in accordance with intensities of multiple rays of reflected light, detected by the at least one detector.

2. The aerosol generating system according to claim 1, wherein
the aerosol generating system further comprises a heater that heats the substrate accommodated in the container, and
the controller controls an operation of the heater in accordance with a determination result as to whether an article inserted in the container is the substrate.

3. The aerosol generating system according to claim 2, wherein
when the controller determines that an article inserted in the container is the substrate, the controller causes the heater to start performing heating.

4. The aerosol generating system according to claim 2, wherein
when the controller determines that an article inserted in the container is the substrate, the controller permits the heater to perform heating, and, when the controller determines that an article inserted in the container is not the substrate, the controller prohibits the heater from performing heating.

5. The aerosol generating system according to any one of claims 1 to 4, wherein
the aerosol generating system includes the two detectors, and
the two detectors are disposed at locations different from each other.

6. The aerosol generating system according to claim 5, wherein
the controller causes each of the two detectors to operate at timing different from each other.

7. The aerosol generating system according to claim 5 or 6, wherein
the controller causes one of the two detectors to operate and the other one of the two detectors to sleep.

8. The aerosol generating system according to any one of claims 5 to 7, wherein
the two detectors are disposed at locations different from each other in an insertion direction of the substrate.

9. The aerosol generating system according to any one of claims 5 to 7, wherein
the two detectors are disposed at locations that at least partially overlap each other in an insertion direction of the substrate.

10. The aerosol generating system according to any one of claims 5 to 9, wherein
the two detectors are disposed apart from each other by a diameter or greater of a thickest part of an article other than the substrate, estimated to be inserted in the container.

11. The aerosol generating system according to claim 10, wherein
the two detectors are disposed at locations at which an angle formed by directions to radiate light is larger than or equal to 90 degrees and smaller than or equal to 270 degrees in a plane orthogonal to an insertion direction of the substrate.

12. The aerosol generating system according to claim 11, wherein
the two detectors are disposed at locations at which an angle formed by directions to radiate light is 180 degrees in a plane orthogonal to the insertion direction of the substrate.

13. The aerosol generating system according to any one of claims 1 to 12, wherein
the controller determines whether an article inserted in the container is the substrate in accordance with intensities of multiple rays of reflected light, detected at different timings by the same detector.

14. The aerosol generating system according to any one of claims 1 to 13, wherein
the aerosol generating system further comprises at least any one of the substrate and an article other than the substrate, estimated to be inserted in the container.

15. An information processing method that is executed by a computer, the information processing method comprising
determining whether an article inserted in a container having an internal space and an opening that communicates the internal space with an outside is a substrate including an aerosol source in accordance with intensities of multiple rays of reflected light, detected by at least one detector that radiates light to the internal space of the container and detects reflected light received.
